# EUROPEAN PATENT APPLICATION

(11) **EP 4 052 647 A1**
(43) Date of publication of application: **07.09.2022**
(21) Application number: 21160118.2
(22) Date of filing: 02.03.2021
(51) Int. Cl.: A61B 5/25, A61B 5/256, A61B 5/257, A61B 5/259, A61B 5/00, A41D 13/12, A61B 5/01, A61F 15/00, A42B 1/046, A42B 1/06

(54) **HYGIENE PROTECTIVE COVER FOR BODILY WORN DEVICES**

(71) Applicant: Oncomfort SA, 1300 Wavre (BE)
(72) Inventor: Tesse, Julien, 1160 Auderghem (BE); Huyghe, Mario, 8792 Desselgem (BE); Toussaint, Clémence, 4210 Burdinne (BE)
(74) Representative: De Clercq & Partners

(57) **Abstract**

A protective hygiene cover (100) for providing a hygienic barrier over a body of a subject (50) which allows conductance of energy to and/or from a bodily surface of the subject (50) in order to take a measurement from or to provide stimulation to the bodily surface, the protective hygiene cover (100) comprising: a hygiene sheet (110) comprising one or more flexible layers, the hygiene sheet having a body-contact side (10) configured for contact with the subject body, and an opposing exterior side (20); and one or more conductive patches (120) each having a body contact side (10) configured for contact with the body, and an opposing exterior side (20), wherein at least one of the conductive patches (120) is an electrically conductive patch, a heat conductive patch or a light conductive patch.

## Description

### Field of the invention

The present invention is in a field of protective covers, in particular for use with a virtual reality or augmented reality visor and/or for use with electrodes, electrical or other contacts for measuring electrical activity or other signals from the human body or stimulating the human body in electrical or other way. It also is in the field of dispensers for such devices.

### Background to the invention

It is important to maintain hygiene between a subject and a wearable device. The wearable device, however, often requires physical access to the body, for instance, to the skin, in order to take measurements, for instance electroencephalogram (EEG) measurements. It is an aim of the present invention to provide a cover that is compatible with existing measurement electrodes but which still maintains a hygiene barrier.

It is also important to maximise hygiene of a wearable device or other setup on the subject while minimising the number of protective covers, environmental waste and time. Solutions need to be easy and secure to set-up, and involve a minimum of steps so there is less chance for contamination of the wearable device, the patient or the user (e.g. healthcare practitioner). It is an aim of the present invention to provide a protective cover serving the wearable device protecting subject, and user exposed to the non-body contact side of the device. In particular a goal is to provide an integrated protective cover so that the correct placement around patient head and on the wearable device is guaranteed. In particular an aim is the protective cover protects substantially all wearable device, not only the part in direct contact with the subject.

Effectiveness of a protective barrier is diminished when the number steps in applying and securing the barrier is increasing. For instance, with a visor device, an eye cupping comes into contact with the skin. A suitable barrier conforming to the eye cupping profile is difficult to mount because the cupping edge is narrow and the shape curves away from planar to accommodate the nose bridge. It is an aim of the present invention to provide a dispenser that reduces the number of steps and provides a more secure application of the barrier.

### Summary of the invention

Provided is a protective hygiene cover (100) for providing a hygienic barrier over a body of a subject (50) which allows conductance of energy to and/or from a bodily surface of the subject (50) in order to take a measurement from or to provide stimulation to the bodily surface, the protective hygiene cover (100) comprising:
- a hygiene sheet (110) comprising one or more flexible layers, the hygiene sheet having a body-contact side (10) configured for contact with the subject body, and an opposing exterior side (20); and
- one or more conductive patches (120) each having a body contact side (10) configured for contact with the body, and an opposing exterior side (20), wherein at least one of the conductive patches (120) is an electrically conductive patch, a heat conductive patch or a light conductive patch.

The protective hygiene cover (100) may further comprise one or more electrical conductors and/or electrical/electronic circuits connected to the one or more conductive patches (120) that is an electrically conductive patch.

At least one of the conductive patches (120) may be permanently attached to the hygiene sheet (110) or is dismountably attached to the hygiene sheet (110).

The protective hygiene cover (100) may be formed as a head garment (300) having at least a scalp-covering portion (310) covering at least a part of the scalp of the subject (50), and having an anterior side (32), a posterior side (34), lateral sides (30, 31), a superior direction (36) and an inferior direction (38).

At least one of the conductive patches (120) may be an electrically conducting patch that is configured for attachment on the exterior side to a skin-placement electrically conducting electrode (200) that is an EEG electrode, ECG electrode, EMG electrode, galvanic electrode, or an electrically-stimulating electrode.

The anterior side (32) of the scalp-covering portion (310) may be provided with a visor portion (320) extending in an inferior direction (38) at least below the eyes of the subject, the visor portion (310) having one or more cut-outs (312) for the eyes.

The head garment head (300) may further comprise a wrap portion (330) extending in an inferior direction from the visor portion (320), the wrap portion (330) configured for placement (by folding) in a superior direction (36) over the visor portion (320) and/or over a visor device (600) placed over the eyes of the subject (50).

The wrap portion (330) may comprise an attachment element (332) configured to attach the wrap portion (330) in position over the visor portion and/or over a visor device placed over the eyes of the subject.

The head garment (300) may further comprise a latero-posterior portion (350) that is an extension of the hygiene sheet (110) in an inferior direction (38) from the scalp-covering portion (310) below the ears of the subject on the lateral (30, 31) and posterior sides (34)..

The head garment (300) may further comprise a respiratory portion (340) that is an inferior (38) extension of the visor portion (320) and covering the nostrils' and mouth of the subject.

The protective hygiene cover (100) may be provided with a securing element and/or adjusting element, configured to secure and/or adjust the protective hygiene cover (100) with respect to the subject.

Further provided is a head garment (300) formed at least partially, preferably entirely from a hygiene sheet (110) comprising one or more flexible layers, the hygiene sheet having a body-contact side (10) configured for contact with the subject body, and an opposing exterior side (20) the head garment (300) comprising:
- a scalp-covering portion (310) at least the scalp of the subject (50), and having an anterior side (32), a posterior side (34), lateral sides, a superior direction (36) and an inferior direction (38),
- a visor portion (320) extending in an inferior direction (38) from the scalp-covering portion (310) at least below the eyes of the subject (50), the visor portion (310) having one or more cut-outs (312) for the eyes, and
- a wrap portion (330) extending in an inferior direction (38) from the visor portion (320), the wrap portion (330) configured for placement in a superior direction (36) over the visor portion (320) and/or over a visor device (600) placed over the eyes of the subject (50).

The head garment (300) may further comprising one or more of the features as described herein.

A dispenser (500) for offering a protective hygiene barrier (90) for mounting by contact to a bodily-worn device (600), wherein:
- the bodily-worn device (600) has an interface part (620) shaped for an abutting contact with a surface of a bodily relief region (52) of a subject,
- the dispenser (500) comprises :
   ∘ a dispensing support (510) having a force-receiving region (512) shaped in relief to complement the device interface part (620) and configured to receive an application force applied by the device interface part (620),
   ∘ a protective hygiene barrier (90) positioned over the force-receiving region (512).
- the dispenser (500) configured to spatially distribute the application force from the interface part (620) to the protective hygiene barrier (90).

The bodily-worn device (600) may comprise a visor device (602), in particular is a VR/AR visor (610), and the interface part (620) is a cupping (622).

The protective hygiene barrier (90) may comprises one or more flexible layers, the protective hygiene barrier (90) having a body contact side (10) configured for contact with the body, and an opposing exterior side (20).

The protective hygiene barrier (90) may a protective hygiene cover (100) as described herein.

The protective hygiene cover (100) may be arranged such that at least a part of the visor portion (320) is positioned over the support relief region (512).

The protective hygiene barrier (90) may be provided with a fixation element configured to attach the protective hygiene barrier (90) to the device interface part (620).

### Figure Legends

**FIG. 1** Depicts a plan view of a conducting hygiene cover.
**FIG. 2A** Depicts a side view of a conducting hygiene cover with a conductive patch and conductive electrode.
**FIG. 2B** Depicts a side view of a conducting hygiene cover with a conductive patch that is conductive electrode that is a standard skin placement electrode.
**FIG. 3** Shows a side view of a head garment having a scalp-covering portion formed from the conducting hygiene cover.
**FIG. 4** Illustrates a direction system employed in the medical arts.
**FIG. 5** Shows a head of a subject.
**FIG. 6** Shows a head garment having a scalp-covering portion and visor portion.
**FIG. 7** Shows a head garment of FIG. 6 and a mounted visor device.
**FIG. 8** Shows a head garment of FIG. 6 and a respiratory portion.
**FIG. 9** Shows a head garment having a scalp-covering portion, visor portion, and wrap portion. The conductive patch may or may not be present.
**FIG. 10** Shows a head garment of FIG. 9 and a mounted visor device. The conductive patch may or may not be present.
**FIG. 11** Shows a head garment of FIG. 10 wherein the wrap portion is folded over the visor device in an active position. The conductive patch may or may not be present.
**FIG. 12** Shows a head garment having a scalp-covering portion and a latero-posterior portion.
**FIG. 12A** Shows the head garment of FIG. 12 provided with a securing element.
**FIG. 13** Shows a head garment of FIG. 12 further having a visor portion, with a split between the visor portion and the a latero-posterior portion.
**FIG. 14** Shows a head garment of FIG. 12 further having a visor portion, in which the visor portion and the latero-posterior portion are joined at the lateral sides.
**FIG. 15** Shows a head garment having a scalp-covering portion, visor portion, and wrap portion and a latero-posterior portion. The conductive patch may or may not be present.
**FIG. 16** Shows a head garment of FIG. 15 and a mounted visor device wherein the wrap portion is in an active position. The conductive patch may or may not be present.
**FIG. 17** Shows a head garment of FIG. 16 further comprising a respiratory portion, and the mounted visor device wherein the wrap portion is in an active position. The conductive patch may or may not be present.
**FIG. 18** Shows a variation of a head garment of FIG. 15 having a split between visor portion-wrap portion and the latero-posterior portion. The conductive patch may or may not be present.
**FIG. 19** Shows a variation of a head garment of FIG. 14 further comprising a respiratory portion. The conductive patch may or may not be present.
**FIG. 20** Shows a head garment and a visor device with further comprising a plurality of fixation straps and an earphone.

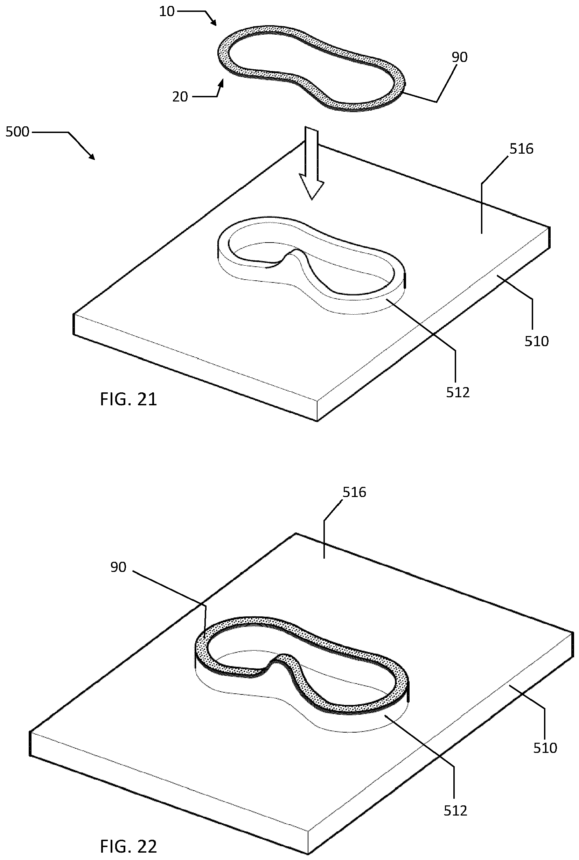

**FIG. 21** Isometric view of a dispenser and a separate a protective hygiene barrier.
**FIG. 22** Depicts the dispenser of FIG. 21, wherein the protective hygiene barrier is mounted on the force-receiving region.
**FIG. 23** Depicts the dispenser of FIG. 22, and a bodily-worn device advancing toward the protective hygiene barrier.
**FIG. 24** Is a side view of the dispenser of FIG. 21 and placement of a protective hygiene barrier.
**FIG. 25** Is a side view of the dispenser of FIG. 21 and a protective hygiene barrier placed over the force-receiving region.
**FIG. 26** Is a side view of the dispenser of FIG. 25 and the visor device advancing toward the force-receiving region.
**FIG. 27** Is a side view of the dispenser of FIG. 26 and the protective hygiene barrier attached to the visor device.

### Detailed description of invention

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. Unless otherwise indicated, all figures and drawings in this document are not to scale and are chosen for the purpose of illustrating different embodiments of the invention. In particular the dimensions of the various components are depicted in illustrative terms only, and no relationship between the dimensions of the various components should be inferred from the drawings, unless so indicated.

It is to be understood that other embodiments may be utilised and structural or logical changes may be made without departing from the scope of the present invention. The following detailed description, therefore, is not to be taken in a limiting sense, and the scope of the present invention is defined by the appended claims.

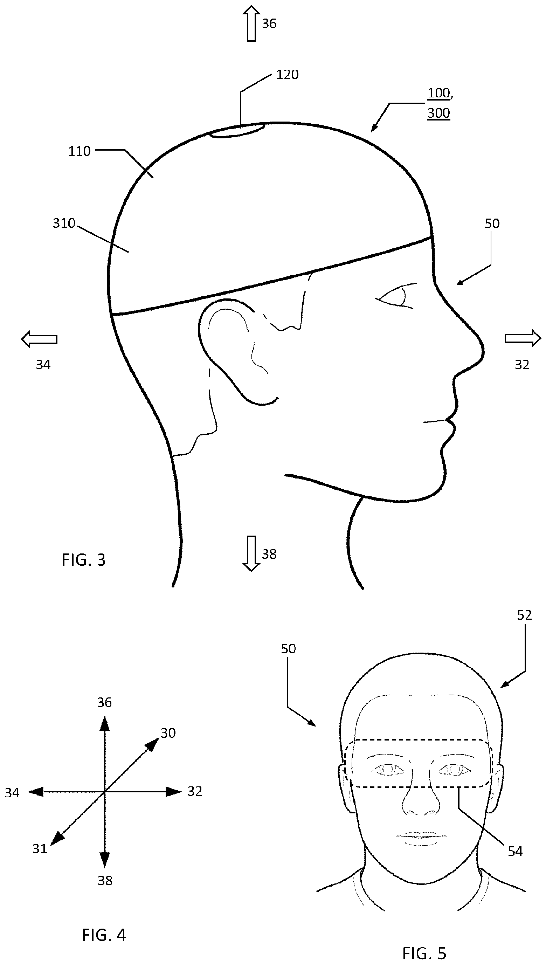

The term "anterior" or "anterior side" (32) refers to a front facing side or direction. The term "posterior" or "posterior side" (34) refers to a rear facing side or direction. The term "lateral" or "lateral side" (31, 32) refers to the sides. The term "superior" or "superior side" (36) refers to an upward side or direction. The term "inferior" or "inferior side" (36) refers to an downward side or direction. Directions are by reference to a human body. Directions are illustrated in **FIGs. 3** and **4****.**

The "subject" refers to a person receiving the hygiene sheet, the conducting hygiene cover, the head garment with or without the conductive patches, or the protective hygiene barrier described herein . The "subject may be the beneficiary of a VR/AR presentation. The "user" refers to a person or persons applying the visor device or bodily-worn device or conducting hygiene cover or head garment to the subject. The user may be a care provider such as a physician, or medical assistant, or non-medical assistant (*e.g*. friend, relative, helper) of the subject. In some circumstances, the user may be the subject.

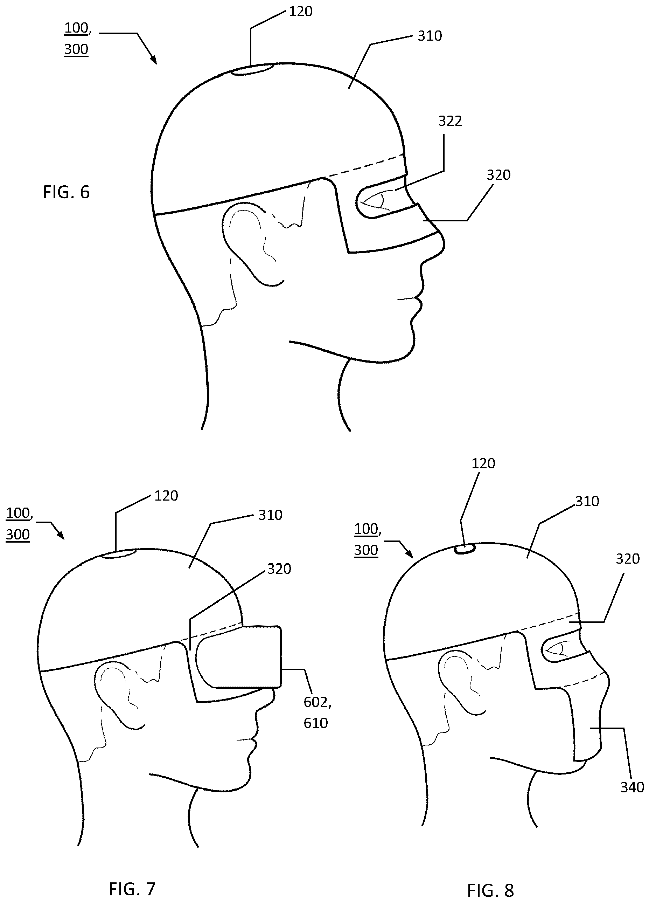

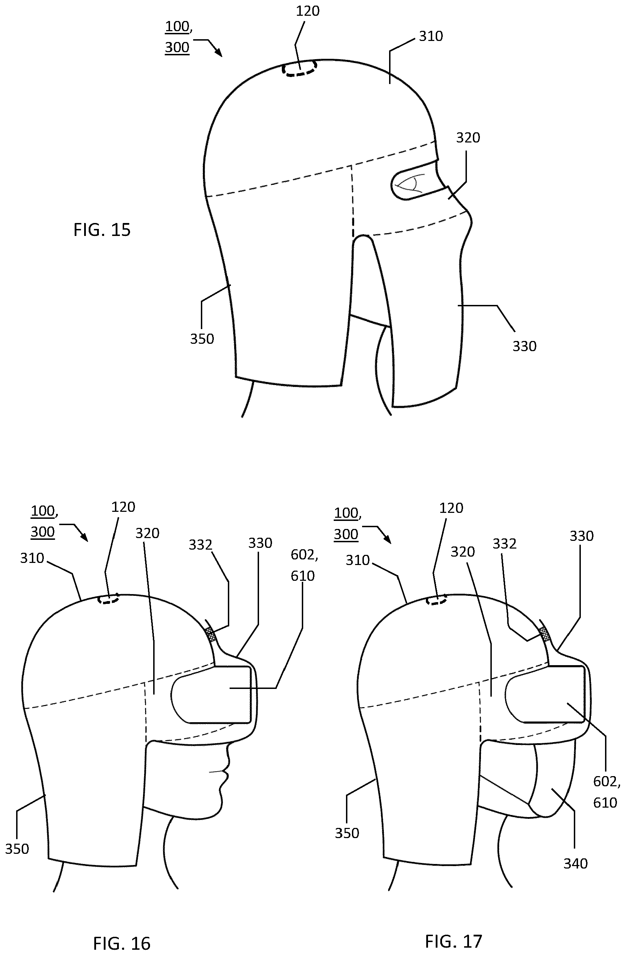

A visor device (602) is a bodily-worn device that covers the eyes of the subject, in particular is a virtual-reality or augmented-reality (VR/AR) visor (610). Examples of a visor device (602) are shown, for instance, in **FIGs. 7****,** **10****,** **11****,** **16****,** **17****,** **20****.**

An VR/AR visor (610) is known in the art as a wearable device that provides virtual and/or augmented and/or mixed reality; which typically includes a display or projector, stereo sound (mono, stereo, multidimensional), and head motion tracking sensors (*e.g*. gyroscopes, accelerometers, structured light systems, etc.). Examples of suitable headsets are those supplied by Oculus *(e.g.* Oculus Rift, Oculus Go), LG electronics *(e.g.* LG 360 VR), HTC (*e.g.* HTC Vive), Samsung (e.g. Samsung Gear VR), Google *(e.g.* Google Cardboard), PICO (e.g. PICO G2 4K).

Visor device (602), in particular the VR/AR visor (610), may be provided with an interface part (620) - a cupping (622) - shaped for an abutting contact with the surface of the face of the subject, in particular around the eyes and bridge of the nose. The cupping (622) may be made at least partially from a compliant material for a more comfortable wearing and for adaptable conformance to the shape of the subject (50).

The visor device (602), in particular the VR/AR visor (610) may include one or more fixation straps (630, a, b, c) configured to fix the visor device (602) or the VR/AR visor (610) to the head of the subject. The one or more fixation straps (630, a, b, c) may be adjustable. The one or more conducting electrodes (200) may be attached to the one or more fixation straps (630, a, b, c). In placing the visor device (602) on the head of the subject (50) over the head garment (300), the one or more fixation straps (630, a, b, c) are positioned so that the one or more integrated conducting electrodes (200) align with the one or more conducting patches (120).

The visor device (602), in particular the VR/AR visor (610) may include one or more sound transducers (640) for emission of sound (e.g. earphone). The one or more sound transducers may be attached to the one or more fixation straps (630, a, b, c).

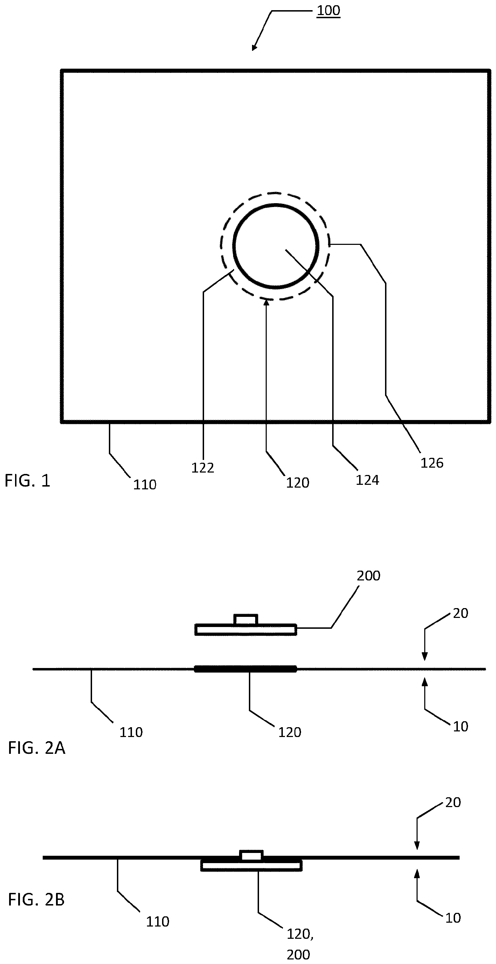

Provided herein is a conducting hygiene cover (100) for providing a protective hygienic barrier over a subject (50) body which allows conductance of energy (e.g. electrical conductance (signals, stimulation, galvanic response), heat, light) to and/or from the bodily surface of the subject (50). The conductance of energy allows measurement from the body surface, or stimulation to the body surface. The energy is conducted through one or more conductive patches (120) configured for conductance of the energy to and/or from the bodily surface of the subject (50). An exemplary conducting hygiene cover (100) is shown in **FIGs. 1,** and **2A** and **2B**.

The conducting hygiene cover (100) comprises a hygiene sheet (110) comprising one or more flexible layers, the hygiene sheet having a body-contact side (10) configured for contact(i.e. full contact or at least partial contact) with the body surface, and an opposing exterior side (20). The conducting hygiene cover (100) further comprises one or more conductive patches (120) each having a body-contact side (10) configured for contact with the body surface, and an opposing exterior side (20). Each conductive patch (120) is formed from a conductive material. The conductive material may be electrically conductive and/or thermally conductive and/or light conductive. Each conductive patch (120) has an exposed region (124).

The subject (50) body refers to a part of the body such as the head (52), limb, chest, scalp, face. The body surface of the subject is typically the skin. The skin may be bare skin or may be at least partially covered with hair. The body surface may be the scalp.

The hygiene sheet (110) is made from any suitable material, such as a material used in disposable medical garments or from a non-disposable material. The disposable material may be sterile. The non-disposable material may be washable and/or sterilisable. The hygiene sheet (110) is non-conductive (i.e. not electrically, or not thermally or not light). It may be made at least partially, substantially or entirely from a material that has a barrier performance regarding penetration of water, and/or blood, and/or any bodily or and/or non-bodily fluid, and/or pathogens transmitted by any of those. The hygiene sheet (110) may be based on polypropylene and/or polyethylene and/or paper. The hygiene sheet may be made from a two layer non-woven fabric. It may be based on polypropylene. It may be based on an elastic fabric (e.g. containing lycra). It is preferably disposable.

The number of conductive patches in the hygiene sheet (110) may be one or more. There may be a plurality (e.g. two, three, four or more) of conductive patches. The conductive patches (120) in the plurality are mutually isolated (e.g. for transfer of electricity, transfer of heat, light), in the hygiene sheet (110).

The conductive patch may be disposed on one side of the hygiene sheet (110) and an exposed region (124) disposed on the other side of the hygiene sheet (110). An opening provided in the hygiene sheet (110), such as a circular opening, may define a boundary of the exposed region (124) on said other side.

The conductive patch may be disposed on one side of the hygiene sheet (110) and the exposed region (124) disposed on the same side of the hygiene sheet (110). A boundary of the exposed region (124) on said one side is defined by the edge (126) of the conductive patch.

The conductive patch may be disposed between one or more layers of the hygiene sheet (110) and the exposed region (124) disposed on one or both sides of the hygiene sheet (110). An opening provided in the hygiene sheet (110), such as a circular opening, may define a boundary of the exposed region (124) on said both sides.

Hence, the exposed region (124) may be on one or both of the body-contact side (10) or opposing exterior side (20) of the conductive patch (120). The opening provided in the hygiene sheet (110), such as a circular opening, may define a boundary of the exposed region (124). Alternatively, the edge (126) of the conductive patch (120) may define a boundary of the exposed region (124).

The conductive patch (120) may be permanently attached to the hygiene sheet (110). The conductive patch (120) may be permanently attached to the hygiene sheet (110) by any process or material such as adhesive (e.g. hot melt), stitching. The conductive patch may be dismountably attached to the hygiene sheet (110). The conductive patch (120) may be dismountably attached to the hygiene sheet (110) by any suitable mechanism such as press-stud, Velcro, pin clip.

The conductive patch (120) may be disposed with a conductive matrix such as a conductive gel or paste one or both of the exterior side (20) or body-contact side (10). The conductive matrix, depending on the formulation, enhances passage of electrical current, electrical signals, heat or light signals. It may assist with adhesion. The conductive matrix may be aqueous. The conductive matrix may be pre-applied to the conductive patch (120); a removeable covering may be provided to reduce dehydration. Alternatively or in addition, the conductive matrix may be applied e.g. by the user prior to use. Examples of conductive matrix include silicone gel (semi-solid), flowable conductive gel or paste such as Nuprep, Elefix conductive EEG paste.

The hygiene sheet (110) may be provided with all one type of conductive patch (e.g. all electrically conductive patch(es), all heat conductive patch(es) or all light conductive patch(es)). The hygiene sheet (110) may be provided with more than two different types of conductive patch (e.g. electrically conductive patch in addition to one or more of heat conductive patch or light conductive patch).

The conductive patch (120) that is an electrically conductive patch may be made from any suitable electrically conductive material. Examples include an electrically conducting metal or a non-metallic electrical conductor. Examples of electrically conducting metals include aluminium or copper. Examples of non-metallic electrical conductors include graphite and conductive polymers. A further example is a composite material containing at least one electrically conductive material. A further example is a type of support (conductive or non-conductive) combined or used with a conductive electrolyte (e.g. porous material with saline solution).

At least one or a plurality of the electrically conductive patches (120) may be configured for attachment to and electrical contact with an electrically conducting electrode (200) on the exterior side (20), and for attachment to and electrical contact with the body surface on the body-contact side (10) (*e.g*. **FIG. 2A**). The electrically conducting electrode (200) may be a standard skin-placement conducting electrode (200). The exterior side of each conductive patch (120) may configured for operative attachment to a conducting electrodes (200). The operative attachment allows the electrically conducting electrode (200) to conduct electrical signals to and/or from the body surface. The operative attachment may be any kind including by contact, by clamping, by a substance having an adhesive effect allowing removal and optional remounting.

At least one or a plurality of the electrically conductive patches (120) may be an electrically conducting electrode (200) (e.g. a standard skin-placement electrode (200)). Such electrically conducting electrode (200) may be attached permanently or dismountably attached to the hygiene sheet (110) (*e.g*. **FIG. 2B*****).***

At least one or a plurality of the electrically conductive patches (120) may each be connected to an electrical conductor (e.g. electrical wires) and/or an electrical/electronic circuit integrated into the hygiene sheet (110).

The electrical conductor carries the electrical current to or from a measurement or stimulation device (*e.g*. electroencephalogram (EEG), electrocardiogram (ECG), electromyography (EMG), electrodermal (EDA), galvanic device or any electrical-stimulation device). The electrical conductor and/or the electronic may run along one surface of the hygiene sheet (110), or run within the hygiene sheet (110), or may be woven into the hygiene sheet (110). The electrical conductor may terminate in a connector measurement or stimulation device, or in an electrical/electronic circuit. The electrical conductor may be flexible.

The electrical/electronic circuit may be attached on one or both surfaces of the hygiene sheet (110), or be disposed within the hygiene sheet (110), or may be woven into the hygiene sheet (110). The electrical/electronic circuit may be connected to one or more electrical conductors (e.g. electrical wires). The electrical/electronic circuit may be connected to one or more electrically conductive patches (120). The electrical/electronic circuit may include a connector for a measurement or stimulation device. The electrical/electronic circuit may be flexible.

The electrically conductive patch (120) may act directly as a measurement/stimulation electrode (e.g. for measuring EEG, ECG, EMG, EDA signals from the human body); it may be electrically connected to the electrical conductor (e.g. electrical wires) and/or an electrical/electronic circuit. Where the electrically conductive patch is a skin placement electrode (e.g. dismountable existing skin-placement electrode (200)) it may be electrically connected to the electrical conductor (e.g. electrical wires) and/or an electrical/electronic circuit.

At least one or a plurality of the conductive patches (120) may be a heat-conductive patch configured for attachment to and heat contact with a temperature sensor (200) on the exterior side (20), and for attachment to and heat contact with the body surface on the body-contact side (10). The exterior side of each heat conductive patch (120) may configured for operative attachment to a temperature sensor (200). The operative attachment allows the temperature sensor (200) to conduct heat to and/or from the body surface. The operative attachment may be any kind including by contact, by clamping, by a substance having an adhesive effect allowing removal and optional remounting.

The conductive patch (120) that is heat conductive may be made from any suitable heat conductive material. Examples include a conducting metal or a non-metallic heat conductor. Examples of heat conducting metals include aluminium or copper. Examples of non-metallic heat conductors include graphite and conductive polymers. A further example is a composite material containing at least one heat conductive material. An electrically conductive patch (120) may also function as a heat conductive patch.

At least one or a plurality of the conductive patches (120) may be a light conductive patch configured for attachment to a light-measuring sensor (200) on the exterior side (20), and for attachment to the body surface on the body-contact side (10). The exterior side of each light conductive patch (120) may configured for operative attachment to a light-measuring sensor (200). The operative attachment allows the light-measuring sensor (200) to conduct light to and/or from the body surface. The operative attachment may be any kind including by contact, by clamping, by a substance having an adhesive effect allowing removal and optional remounting.

The light conductive patch (120) that is light conductive may be made from any suitable heat conductive material. Examples include a light conducting polymer such as acrylic (polymethlamethacrylate), butyrate (cellulose acetate butyrate), lexan (polycarbonate), or PETG (glycol modified polyethylene terphthalate).

The electrically conducting electrode (200) may be any kind of electrode used for transmission of electrical signals to and/or from the body. Examples include electroencephalogram (EEG) electrode, electrocardiogram (ECG) electrode, electromyography (EMG) electrode, electrodermal (EDA) electrode, galvanic electrode or any electrically-stimulating electrode. The electrode may be made from any suitable conducting material including, for instance, gold, silver, silver-silver chloride, or conductive polymer, stainless steel, copper. Medical electrodes are often made of gold, silver or conductive polymer. Silver-silver chloride (Ag-AgCI) electrodes are often used for medical EEG electrodes.

The electrically conducting electrode (200) may be disposed with a conductive matrix such as a conductive gel or paste. The conductive matrix enhances passage of electrical signals. It may assist with adhesion. The conductive matrix may be aqueous. The conductive matrix may be pre-applied to the conducting electrode (200); a removeable covering may be provided to reduce dehydration. Alternatively or in addition, the conductive matrix may be applied *e.g*. by the user prior to use. Examples of conductive matrix include silicone gel (semi-solid), flowable conductive gel or paste such as Nuprep, Elefix conductive EEG paste.

The temperature sensor (200) may be any kind of sensor used for detection of heat from the body. Examples include thermometers, thermocouple sensors or thermisistors.

The temperature sensor (200) may be disposed with a conductive matrix such as a heat conductive gel or paste. The heat conductive matrix enhances passage of heat. It may assist with adhesion. The heat conductive matrix may be aqueous. The heat conductive matrix may be pre-applied to the heat conducting electrode (200); a removeable covering may be provided to reduce dehydration. Alternatively or in addition, the heat conductive matrix may be applied e.g. by the user prior to use. Examples of heat conductive matrix include silicone gel (semi-solid).

The light sensor (200) may be any kind of sensor used for detection of light from the body. Examples include infrared (IR) sensors, or photoplethysmogram (PPG) sensors for measuring heart rate or cardiac cycle or breathing or blood pressure or oxygen saturation in the blood.

The light sensor (200) may be disposed with a light conductive matrix such as a clear light conductive gel. The light conductive matrix facilitates the passage of light. It may assist with adhesion. The light conductive matrix may be aqueous. The light conductive matrix may be pre-applied to the light conducting sensor (200); a removeable covering may be provided to reduce dehydration. Alternatively or in addition, the light conductive matrix may be applied e.g. by the user prior to use. Examples of light conductive matrix include silicone gel (semi-solid).

The conducting hygiene cover (100) may be formed as a head garment (300) having at least a scalp-covering portion (310) covering at least a part, preferably all of the scalp of the subject (50). Provided herein hence is a head garment (300) formed at least partially, preferably entirely from the conducting hygiene cover (100). Several examples of a head garment (300) are shown in **FIGs. 3****,** **6** to **20.** The head garment (300) may be provided in different sizes.

The scalp-covering portion (310) may be shaped to complement the form of the scalp. The body-contact side (10) may have a generally concave shape. It may be elasticated. It may be formed from an elasticated (stretchable) material. It may be provided in different sizes.

The anterior side (32) of the scalp-covering portion (310) may be provided with a visor portion (320). The visor portion (320) of the hygiene sheet (110) extends in an inferior direction (38) at least below the eyes of the subject. It may or may not extend beyond the nose. The visor portion (310) is disposed with one or more cut-outs (312) for the eyes. The exterior side (20) of the visor portion (310) is configured to contact a visor device (602) and to provide a hygiene-protective barrier to the body surface. Several examples of a head garment (300) comprising a visor portion (320) are shown in **FIGs. 6** to **11****,** **13** to **20**.

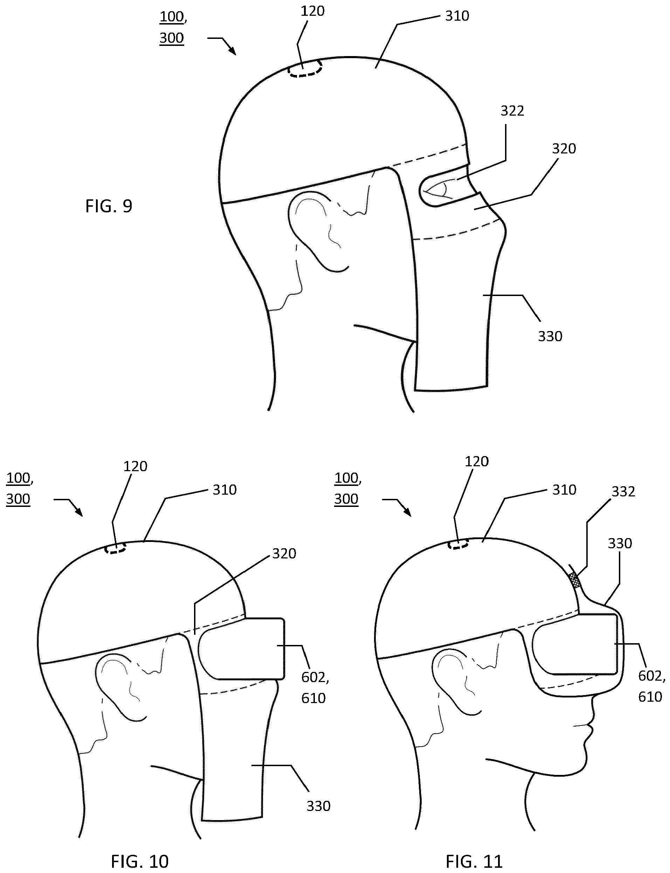

The head garment (300) may further comprise a wrap portion (330) that is an extension of the hygiene sheet (110) in an inferior direction (38) from the visor portion (320). The wrap portion (330) is configured for placement (by folding) in a superior direction (36) over the visor portion (320) and/or over a visor device (602) placed over the eyes of the subject. A length of the wrap portion (330) is sufficient to cover the visor device. The wrap portion (330) protects at least back side of the visor device (602), preferably also the lateral sides. Several examples of a wrap portion (330) are shown in **FIGs. 9** to **11****,** **15** to **19**.

The wrap portion (330) has two principle operational positions. An initial position extends in an inferior direction (38) used while the visor device (602) is being mounted on the subject. An active position where it is folded in a superior direction (36) and covers the visor portion (320) and/or over a visor device (602) placed over the eyes of the subject. It extends hygiene protection to the visor portion (320) and/or visor device (602) for protection of both the subject and user.

It is appreciated that a fixed boundary between the visor portion (320) and the wrap portion (330) does not need to be introduced. The boundary can change depending on the size of the visor device (602). The visor portion (320) and the wrap portion (330) may be formed from a continuous sheet of hygiene sheet (110).

The wrap portion (330) may comprise an attachment element (332) configured to attach the wrap portion (330) in position over the visor portion (320) and/or over the visor device (602) placed over the eyes of the subject. The attachment element (332) attaches the wrap portion (330) to the scalp portion (310) of the head garment. The attachment element may be, for instance, an adhesive strip, a peel away adhesive strip, a Velcro fastener, a pin, an elastic loop. The attachment element (332) facilitates the protective role, and avoids a need for a separate cover for the visor device (602). It reduces waste including from packaging. Several examples of an attachment element (332) are shown in **FIGs. 11****,** **16, 17****.**

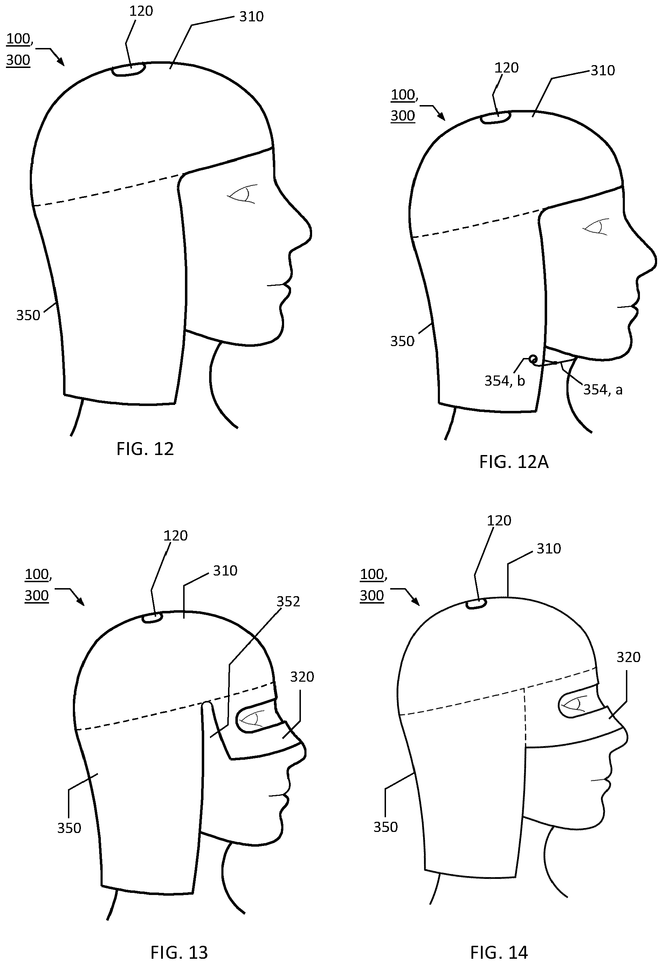

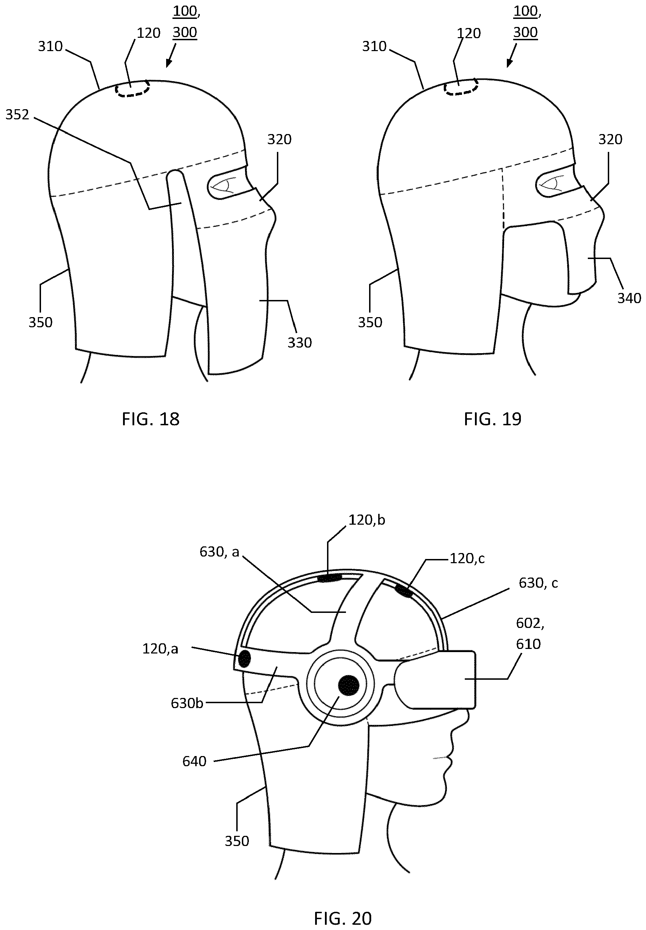

The head garment (300) may further comprise a latero-posterior portion (350) that is an extension of the hygiene sheet (110) in an inferior direction (38) from the scalp-covering portion (310) below the ears of the subject on the lateral (30, 31) and posterior sides (34). Several examples of a latero-posterior portion (350) are shown in **FIGs. 12** to **19****.** The latero-posterior portion (350) may be attached laterally to the visor portion (320) (e.g. **FIGs. 14** to **17****,** **19**) or may be separated by a gap (352) (*e.g*. **FIGs. 13****,** **18**)**.** The latero-posterior portion (350) reduces contact between the body and a support such as a bed or chair.

The head garment (300) may further comprise a respiratory portion (340) that is an extension of the visor portion (320) and covering the nostrils' and mouth of the subject. The respiratory portion (340) is disposed on an anterior side (32) of the head garment and extends in an inferior direction (38) The respiratory portion (340) is configured to provide a hygiene protection against inhalation and/or exhalation of particles (e.g. microbial particles, dust). The respiratory portion (340) allows an additional functionality of the head garment (300), and avoids a need for a separate face mask. It is easier to setup, better protection, and prevent downsides of using a separate face mask with a visor device (*e.g*. condensation in the optics). It reduces waste including from packaging. The respiratory portion (340) may be made from the same or different material as the hygiene sheet (110). It may be made from any material with having sufficient bacterial filtration efficiency, and/or particulate filtration efficiency, and/or fluid resistance and/or pressure resistance. Several examples of respiratory portion (340) are shown in **FIGs. 8****,** **17****,** **19****.**

The head garment (300) may be provided with a securing element and/or adjusting element, configured to secure and/or adjust the protective hygiene cover (100) with respect to the subject. The securing element may be a Velcro fastener, pin press-stud fastener, fastening tie, button-hole fastener, elasticated seam or loop, adhesive strip, a peel away adhesive strip, a cord that can be pulled tight and optionally knotted (*e.g*. under the subject chin). The latero-posterior portion may comprise a attachment element for securing the hygiene protection on the patient's head, ensuring that it does not displace. The latero-posterior portion (350) may comprise a securing element for adjusting the hygiene protection to the patient head. **FIG. 12A** shows an example of an untightened securing element (354) comprising a cord (354) and eyelet (354) provided on the latero-posterior portion (350).

Provided herein is a head garment (300) formed at least partially, preferably entirely from the hygiene sheet (110) having:
- a scalp-covering portion (310) covering at least a part, preferably all of the scalp of the subject (50),
- a visor portion (320) extending in an inferior direction (38) at least below the eyes of the subject, and
- a wrap portion (330) extending in an inferior direction from the visor portion (320).

The head garment (300) may further comprise one or more of a latero-posterior portion (350), a respiratory portion (340), securing element and/or adjusting element. The hygiene sheet (110), scalp-covering portion (310), the visor portion (320), the wrap portion (330), latero-posterior portion (350), respiratory portion (340), and securing element and/or adjusting element are as described elsewhere herein. The head garment is provided without conductive patches allowing hygienic mounting of the visor device (602), in particular the AR/VR visor (610) to the subject (50), and hygienic protection of a back-side of the visor device (602, 610). It avoids a need for a separate cover for the visor device (602). It reduces waste including from packaging. It avoids excessive cleaning of the visor device (602, 610), and allow a hygienic manual adjustment by the user while worn *in situ.* Several examples of such a head garment (300) are shown in **FIGs. 9, 10****,** **15** to **17, 18, 19** wherein the conductive patch or patches (120) are absent.

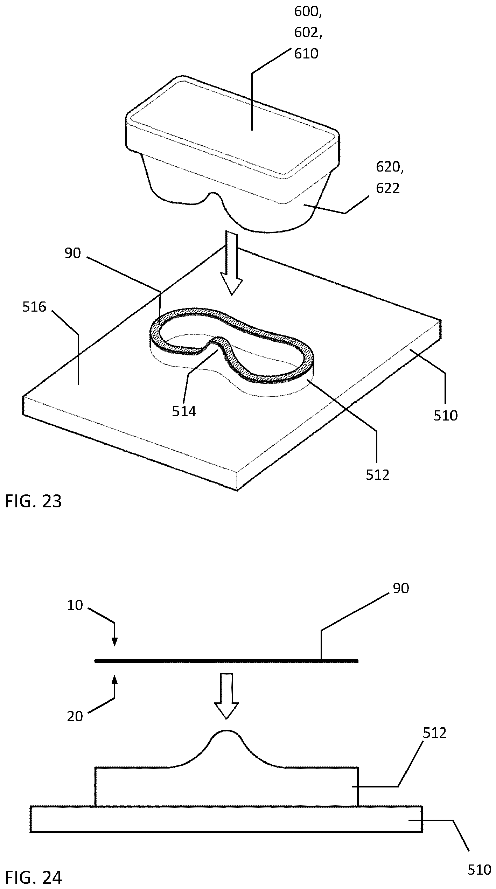

Further provided herein is a dispenser (500) for offering a protective hygiene barrier (90) for mounting by contact to a bodily-worn device (600). Several examples of a dispenser (500) are shown in **FIGs. 23 to 26****.** The dispenser (500) allows easier attachment of the device to the hygiene protection and ensures a good fit between the two parts.

The bodily-worn device (600) has an interface part (620) shaped for an abutting contact with a surface of a bodily relief region (54) of a subject (50). The bodily relief region (52) is a part of the body having raised and/or sunken parts compared with a plane. Examples of a bodily relief region (54) include the region around the around the eyes including the bridge of the nose, or a region on a limb. A bodily relief region (54) around the eyes is shown in **FIG. 5****.** An example of an interface part (620) is an eye cupping (622), for instance of the type found in safely goggles or in a VR/AR visor. An example of an eye cupping (622) is shown in **FIG. 23****.**

The dispenser (500) comprises a dispensing support (510) having a force-receiving region (512) shaped in relief to complement the device interface part (620) and being configured to receive an application force applied by the device interface part (620). The application of force may be downward. The force-receiving region (512) may be shaped in relief meaning it has raised and/or sunken parts compared with a remainder (516) of the dispensing support. It may have a structure of a raised looped ridge, with a further raised structure (514) accommodating the nose bridge as shown, for instance, in **FIG. 23****.** The raised looped ridge complements an eye cupping (622). The remainder (516) of the force-receiving region (512) may be planar, curved, or adopt a form that offers the force-receiving region (512) for contact with the bodily-worn device (600) interface part (620).

The force-receiving region (512) is bend resistive. It may not yield or yield only partially on the application of force. The force-receiving region (512) may be made from any suitable material, such as a polymer (e.g. polyethylene, polycarbonate, cardboard). It may be formed by a moulding process.

The dispensing support (510) is bend resistive. It may not yield or yield only partially on the application of force. The dispensing support (510) may be made from any suitable material, such as a polymer (e.g. polyethylene, polycarbonate, cardboard). It may be formed by a moulding process.

The force-receiving region (512) and the dispensing support (510) may be formed from the same material. The force-receiving region (512) and the dispensing support (510) may be formed as one piece, for instance, by moulding or additive manufacturing (3D printing).

The dispenser (500) may further comprise a protective hygiene barrier (90) mounted on or positioned over the force-receiving region (512). The protective hygiene barrier (90) comprises one or more flexible layers, the protective hygiene barrier (90) having a body contact side (10) configured for contact with the body, and an opposing exterior side (20). The protective hygiene barrier (90) may be provided with a fixation element configured to attach the protective hygiene barrier (90) to the device interface part (620). The fixation element may be disposed on the exterior side (20) of the protective hygiene barrier (90). The fixation element may be an adhesive layer or one part of a Velcro fastener. The dispenser (500) may be provided with the protective hygiene barrier (90) pre-mounted on the force-receiving region (512). The conductive hygiene cover (100) may be attached to the force-receiving region by an adhesive less strong than the fixation element.

The protective hygiene barrier (90) may be the conductive hygiene cover (100), in particular the head garment (300), or the head garment (300) without conductive patches (e.g. with wrap portion (330)) as described elsewhere herein. The head garment (300) may be arranged such that at least a part of the visor portion (320), in particular surrounding the cut out for the eyes (322), is mounted on or positioned over the support relief region (512). The remainder of the conductive hygiene cover (100), in particular of the head garment (300), may be gathered or folded to reduce the amount of space it occupies on the dispensing support (510). It may be gathered or folded in such a way to allow easy positioning around the subject head.

The dispensing support (510) may be provided with a manual gripping region, which is a region clear of the protective hygiene barrier (90), allowing the dispensing support (510) to be manually handled without contamination of the protective hygiene barrier (90). It may be an underside or a side edge of the dispensing support (510).

The dispenser (500) reduces amount of manual interaction during attachment of the protective hygiene barrier (90). The shape of the interface part (620) makes it difficult to attach by conventional means. In particular when the protective hygiene barrier (90) is the conductive hygiene cover (100) to be attached to a looped ridge as shown in FIG. 23, alignment requires a high degree of manual dexterity and patience. The protective hygiene barrier (90) is placed in an orientation and in relief form that allows a one-step application.

The dispenser (500) is configured to spatially distribute the application force from the interface part (620) to the protective hygiene barrier (90). Advantageously the force-receiving region (512) is shaped to complement the device interface part (620) thereby spreading the application force. This avoids an uneven mounting which could result in a detachment of the protective hygiene barrier (90) from the bodily-worn device, or in gaps where particles could infiltrate. This is particularly the case around the nose bridge.

The bodily-worn device (600) may be a visor device (602), in particular it may be a VR/AR visor (610). The visor device (602) is any that covers the eyes and includes safety goggles; virtual-reality or augmented-reality (VR/AR) visor (610) as described elsewhere herein.

The dispenser (500) may be provided with a cover/and or disposed in clean packaging.

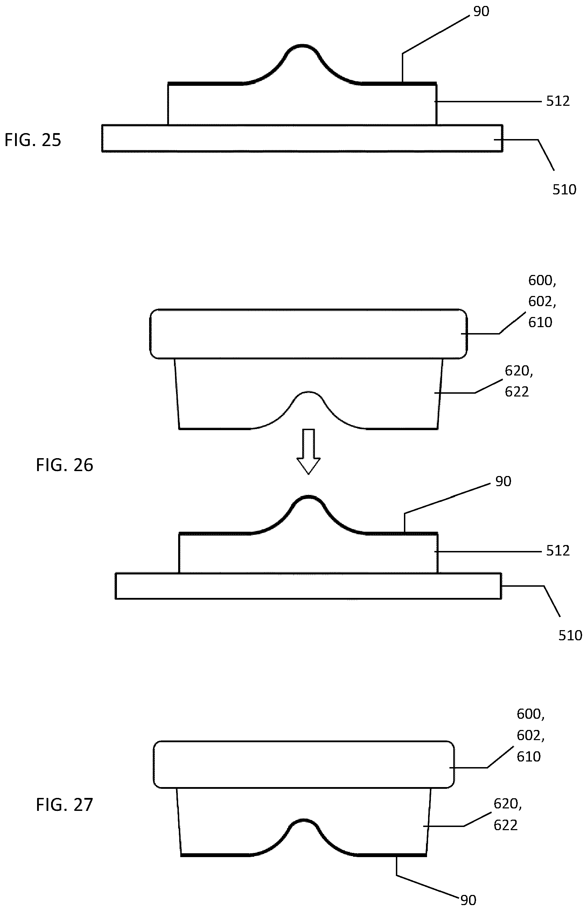

**FIGs. 24** to **26** show a sequence of steps for applying the protective hygiene barrier (90) to the bodily-worn device (600), that is a visor device (602) namely a VR/AR visor (610) using the dispenser (500). In **FIG. 24****,** the protective hygiene barrier (90) is applied to the force-receiving region (512) of the dispensing support (510) resulting in the protective hygiene barrier (90) being mounted on the force-receiving region (512). This is typically done during manufacture. The VR/AR visor (610) is advanced to the force-receiving region (512) (**FIG. 26**)**.** After contact and applying application force, the protective hygiene barrier (90) is transferred to the VR/AR visor (610) (**FIG. 27**)

## Claims

1. A protective hygiene cover (100) for providing a hygienic barrier over a body of a subject (50) which allows conductance of energy to and/or from a bodily surface of the subject (50) in order to take a measurement from or to provide stimulation to the bodily surface, the protective hygiene cover (100) comprising:
- a hygiene sheet (110) comprising one or more flexible layers, the hygiene sheet having a body-contact side (10) configured for contact with the subject body, and an opposing exterior side (20); and
- one or more conductive patches (120) each having a body contact side (10) configured for contact with the body, and an opposing exterior side (20), wherein at least one of the conductive patches (120) is an electrically conductive patch, a heat conductive patch or a light conductive patch.

2. The protective hygiene cover (100) according to claim 1, further comprising one or more electrical conductors and/or electrical/electronic circuits connected to the one or more conductive patches (120) that is an electrically conductive patch.

3. The protective hygiene cover (100) according to claim 1 or 2, wherein at least one of the conductive patches (120) is permanently attached to the hygiene sheet (110) or is dismountably attached to the hygiene sheet (110).

4. The protective hygiene cover (100) according to any one of the previous claims formed as a head garment (300) having at least a scalp-covering portion (310) covering at least a part of the scalp of the subject (50), and having an anterior side (32), a posterior side (34), lateral sides (30, 31), a superior direction (36) and an inferior direction (38).

5. The protective hygiene cover (100) according to claim 4, wherein at least one of the conductive patches (120) is an electrically conducting patch that is configured for attachment on the exterior side to a skin-placement electrically conducting electrode (200) that is an EEG electrode, ECG electrode, EMG electrode, galvanic electrode, or an electrically-stimulating electrode.

6. The protective hygiene cover (100) according to claim 4 or 5, wherein the anterior side (32) of the scalp-covering portion (310) is provided with a visor portion (320) extending in an inferior direction (38) at least below the eyes of the subject, the visor portion (310) having one or more cut-outs (312) for the eyes.

7. The protective hygiene cover (100) according to claim 6, wherein head garment head (300) further comprises a wrap portion (330) extending in an inferior direction from the visor portion (320), the wrap portion (330) configured for placement (by folding) in a superior direction (36) over the visor portion (320) and/or over a visor device (600) placed over the eyes of the subject (50).

8. The protective hygiene cover (100) according to claim 7, wherein the wrap portion (330) comprises an attachment element (332) configured to attach the wrap portion (330) in position over the visor portion and/or over a visor device placed over the eyes of the subject.

9. The protective hygiene cover (100) according to any of claims 4 to 8, wherein the head garment (300) further comprises a latero-posterior portion (350) that is an extension of the hygiene sheet (110) in an inferior direction (38) from the scalp-covering portion (310) below the ears of the subject on the lateral (30, 31) and posterior sides (34).

10. The protective hygiene cover (100) according to any of claims 6 to 9, wherein the head garment (300) further comprises a respiratory portion (340) that is an inferior (38) extension of the visor portion (320) and covering the nostrils' and mouth of the subject.

11. The protective hygiene cover (100) according to any of claims 1 to 10, provided with a securing element and/or adjusting element, configured to secure and/or adjust the protective hygiene cover (100) with respect to the subject.

12. A head garment (300) formed at least partially, preferably entirely from a hygiene sheet (110) comprising one or more flexible layers, the hygiene sheet having a body-contact side (10) configured for contact with the subject body, and an opposing exterior side (20) the head garment (300) comprising:
- a scalp-covering portion (310) at least the scalp of the subject (50), and having an anterior side (32), a posterior side (34), lateral sides, a superior direction (36) and an inferior direction (38),
- a visor portion (320) extending in an inferior direction (38) from the scalp-covering portion (310) at least below the eyes of the subject (50), the visor portion (310) having one or more cut-outs (312) for the eyes, and
- a wrap portion (330) extending in an inferior direction (38) from the visor portion (320), the wrap portion (330) configured for placement in a superior direction (36) over the visor portion (320) and/or over a visor device (600) placed over the eyes of the subject (50).

13. The head garment (300) according to claim 12, further comprising one or more of the features of any one of claims 9 to 11.

14. A dispenser (500) for offering a protective hygiene barrier (90) for mounting by contact to a bodily-worn device (600), wherein:
- the bodily-worn device (600) has an interface part (620) shaped for an abutting contact with a surface of a bodily relief region (52) of a subject,
- the dispenser (500) comprises :
∘ a dispensing support (510) having a force-receiving region (512) shaped in relief to complement the device interface part (620) and configured to receive an application force applied by the device interface part (620),
∘ a protective hygiene barrier (90) positioned over the force-receiving region (512).
- the dispenser (500) configured to spatially distribute the application force from the interface part (620) to the protective hygiene barrier (90).

15. The dispenser (500) according to claim 14, wherein the bodily-worn device (600) comprises is a visor device (602), in particular is a VR/AR visor (610), and the interface part (620) is a cupping (622).

16. The dispenser (500) according to claim 14 or 15, wherein the protective hygiene barrier (90) comprises one or more flexible layers, the protective hygiene barrier (90) having a body contact side (10) configured for contact with the body, and an opposing exterior side (20).

17. The dispenser (500) according to any one of claims 14 to 16, wherein the protective hygiene barrier (90) is a protective hygiene cover (100) according to any one of claims 1 to 11.

18. The dispenser (500) according to claim 17, wherein the protective hygiene cover (100) is a arranged such that at least a part of the visor portion (320) is positioned over the support relief region (512).

19. The dispenser (500) according to claim 18, wherein the protective hygiene barrier (90) is provided with a fixation element configured to attach the protective hygiene barrier (90) to the device interface part (620).
